Europäisches Patentamt

European Patent Office

Office européen des brevets

(19)

(11) Publication number: **0 369 013 B1**

# (12) EUROPEAN PATENT SPECIFICATION

(45) Date of publication of patent specification: **06.10.93**   (51) Int. Cl.⁵: **G01N 31/22**

(21) Application number: **88906132.1**

(22) Date of filing: **26.07.88**

(86) International application number:
**PCT/JP88/00748**

(87) International publication number:
**WO 89/01151 (09.02.89 89/04)**

(54) **TESTING PIECE AND TESTING DEVICE FOR MEASURING CHLORIDE.**

(30) Priority: **31.07.87 JP 190394/87**

(43) Date of publication of application:
**23.05.90 Bulletin 90/21**

(45) Publication of the grant of the patent:
**06.10.93 Bulletin 93/40**

(84) Designated Contracting States:
**BE CH DE FR GB IT LI NL SE**

(56) References cited:
**EP-A- 0 189 809**
**JP-A- 5 984 158**
**JP-A-60 177 266**
**US-A- 3 447 904**
**US-A- 4 163 039**

**PATENT ABSTRACTS OF JAPAN, vol. 9, no. 180 (C-293)[1903], 25th July 1985; & JP-A-60 49 073**

(73) Proprietor: **TERUMO KABUSHIKI KAISHA**
**No. 44-1, Hatagaya 2-chome,**
**Shibuya-ku**
**Tokyo 151(JP)**

(72) Inventor: **MORIKAWA, Naoki Terumo**
**Kabushiki Kaisha**
**1727-1, Tsukijiarai**
**Showa-cho**
**Nakakoma-gun**
**Yamanashi 409-38(JP)**
Inventor: **SUZUKI, Hiroshi Terumo Kabushiki**
**Kaisha**
**44-1, Hatagaya 2-chome**
**Shibuya-ku**
**Tokyo 151(JP)**

(74) Representative: **Gillard, Marie-Louise et al**
**Cabinet Beau de Loménie**
**158, rue de l'Université**
**F-75340 Paris Cédex 07 (FR)**

**Description**

Technical Field

The present invention relates to a novel test strip and a test device having said strip fixed thereon for the measurement of a chloride.

The test strip and the test device according to the invention are utilized for the determination of a chloride, for example, sodium chloride, in a liquid specimen such as plasma, transfusion and urine.

Background Art

One of the known methods for the determination of a chloride or a chlorine ion is the Volhard method, according to which a predetermined excess amount of silver nitrate is added to a sample solution containing a chlorine ion to form white precipitates of silver chloride, which is separated by filtration, the silver ion remaining in excess in the filtrate is back titrated with a thiocyanate solution using $Fe^{3+}$ as an indicator, and concentration of the chloride is calculated on the basis of the consumed amount of the thiocyanate.

Whereas the method enables precise measuring the concentration of a chloride, it has such a disadvantage as requiring a titration apparatus as well as a complicated titration operation. In this respect, a much simpler method for measuring the concentration of a chloride in specimens has been desired.

Disclosure of the Invention

It is an object of the invention to provide a test strip and a test device capable of measuring the concentration of a chloride without titration operation, that is, simply by immersing same in a test solution.

According to the invention, there are provided the following test strip and test device for the measurement of a chloride.

1) A test strip for the measurement of a chloride comprising a carrier on which a water-soluble thiocyanate is carried, a separation-membrane layer mounted on the upper surface of said carrier and a water-soluble silver-salt and color-indicator layer mounted on the upper surface of said separation-membrane layer, said separation-membrane layer being capable of preventing the permeation of the water-soluble silver salt and the color indicator when they are in dry and solid state and allowing the silver salt and the color indicator to permeate when they are in ionized or dissolved state.

2) A test strip according to item 1 wherein the carrier is a filter paper.

3) A test strip according to item 1 or 2 wherein said water-soluble silver-salt and color-indicator layer is composed of a water-soluble silver salt layer and a color-indicator layer.

4) A test strip according to any one of items 1-3 wherein said water-soluble thiocyanate is soluble in an organic solvent.

5) A test strip according to any one of items 1-4 wherein said separation membrane layer comprises substantially a macromolecular compound.

6) A test strip according to item 5 wherein the macromolecular compound is hydroxypropylmethylcellulose or methylcellulose.

7) A test device for the measurement of a chloride comprising a support and two or more test strips fixed on said support which comprise a carrier carrying a water-soluble thiocyanate and a separation-membrane layer mounted on the upper surface of said carrier and a water-soluble silver-salt and color indicator layer mounted on the upper surface of said separation-membrane layer, said separation-membrane layer being capable of preventing the permeation of the water-soluble silver salt and the color indicator when they are in dry and solid state and allowing the water-soluble silver salt and the color indicator to permeate when they are in ionized or dissolved state and a concentration of the water-soluble silver salt and/or the water-soluble thiocyanate in the test strips being different one another.

As a chloride-containing specimen to be measured according to the invention are mentioned, for example, plasma, plasma substitutions, amino acid and/or vitamin-containing transfusions, fruit juice, wine, beer, soup, miso soup, food extracts, urine and other liquid test materials. As examples of the chloride are mentioned sodium chloride, potassium chloride, magnesium chloride, calcium chloride and the like.

Measurement of these chlorides is usually carried out for the assay of components of various pharmaceutical preparations.

The constitution and the method for the preparation of the test strip for the measurement of a chloride according to the invention will be described.

As mentioned above, the test strip for the measurement of a chloride comprises the water-soluble silver-salt and color-indicator layer put via the separation-membrane layer on the carrier carring a water-soluble thiocyanate.

As the carrier for the water-soluble thiocyanate are preferably used filter papers, papers, unwoven cloths, woven cloths, knitted goods, ceramics, glass fibers and the like. There may be employed any of those materials which are not reactive with the water-soluble thiocyanate and other reagents used in the test strip and are capable of carrying the water-soluble thiocyanate. Filter papers are particularly preferable. The thickness is preferably of 150 to 500 $\mu$m, but not limited thereto.

As the water-soluble thiocyanate may be used ammonium thiocyanate, sodium thiocyanate, potassium thiocyanate and the like. In order to carry a water-soluble thiocyanate on a carrier, the water-soluble thiocyanate is dissolved in a solvent, the carrier is immersed in the solution and the carrier impregnated with the water-soluble thiocyanate is dried. It is preferred to set concentration of $SCN^-$ ion to be lower than the concentration of $Ag^+$ below-mentioned because the color reaction is negative if the chloride concentration is lower than the detectable limit. The solvent is preferably water or acetone.

To the thiocyanate solution is desirably added a fixing agent such as sodium alginate.

The separation-membrane layer is employed for successively effecting a primary reaction between $Cl^-$ and $Ag^+$ and a secondary reaction between $Ag^+$ and $SCN^-$. For this purpose, the separation-membrane layer must prevent the permeation of the water-soluble silver salt and the color indicator when they are in dry and solid state and allow, when immersing test strips in a test solution, the silver salt and the color indicator to permeate when they are ionized or dissolved.

Suitable material constituting such separation-membrane layer is a semipermeable macromolecular compound, which is preferably soluble in an organic solvent for a desirable operation in forming said layer. Illustrative are polyvinylpyrrolidone, methylcellulose, hydroxypropylcellulose, hydroxypropylmethylcellulose, polyvinyl alcohol, polyethylene oxide, cellulose acetate, ethylcellulose, polyvinyl formal, polybutadiene, polymethyl methacrylate, polyvinyl butyral, styrene, maleic anhydride copolymers and the like. Methylcellulose, hydroxypropylcellulose, cellulose acetate and the like are preferred because the above-mentioned thiocyanates are insoluble in an organic solvent used for the above materials.

The separation-membrane layer is formed by dissolving the above-mentioned macromolecular compound in an organic solvent in which water-soluble thiocyanates are insoluble, immersing in the solution the above-mentioned thiocyanate-impregnated carrier and drying the product thus produced. As a suitable organic solvent are mentioned methylene chloride, benzene or dichloroethane and mixtures thereof.

Upon the upper surface of the separation-membrane layer is put a layer comprising a water-soluble silver salt and a color indicator. The latter layer is formed by dissolving the water-soluble silver salt and the color indicator in an organic solvent in which the macromolecular compound used for the formation of separation-membrane layer is insoluble, immersing in the solution a carrier on which the above-mentioned separation-membrane layer has been formed and drying the product thus produced.

As described above, it is required that the solvents used in a forming step of respective layers on the test strip of the invention should be one in which components of each of the adjacent layers are insoluble. The water-soluble silver salt includes silver nitrate, silver acetate, silver malonate, silver perchlorate and the like, silver nitrate being preferable.

Concentration of the solution of a water-soluble silver salt is determined after fixation of the amounts of $Ag^+$ ions and $SCN^-$ ions to be contained in the test strips. As the color indicator may be employed a ferric ion-containing salt used in the Volhard method such as ferric nitrate or ammonium ferric sulfate. A combination of a water-soluble cobalt (II) compound and a color sensitizer which we have discovered may also be employed. Such color indicator will change from pink to blue when reacted with a thiocyanate ion.

As the water-soluble cobalt (II) compound may be used cobalt (II) sulfate, cobalt (II) acetate, cobalt (II) nitrate and the like.

As the color sensitizer are mentioned quaternary ammonium salts, methanol, ethanol, acetone, polyvinylpyrrolidone and tributyl phosphate. The quaternary ammonium includes aliphatic quaternary ammonium salts such as tetraalkyl ammonium salts containing four or more carbon atoms, for example, tetrabutyl ammonium nitrate, tetrabutylammonium perchlorate and tetrabutylammonium borofluoride, and in addition, aromatic quaternary ammonium salts, heterocyclic quaternary ammonium salts, and the like.

Such color sensitizer is preferably present in an amount so as to provide a molar concentration equal to that of $Co^{2+}$ ion.

In addition to the above, a discoloration-preventing agent such as polyacrylic acid may be used.

The silver nitrate and the color indicator may be divided into two groups which are respectively dissolved in different solvents to prepare two solutions and successively form two layers.

3

A test device, the second invention, has two or more of the above-described test strips having different concentrations of the silver nitrate.

Materials for the support is preferably plastics and the like.

The test strips are fixed on the support in order of increasing (or decreasing) concentration from one end so that chloride concentration in the specimens can semi-quantitatively be determined from the number of color developed-test strips.

Next, an example of the color reaction in the quantitative determination of chlorides using the test strip of the invention will be described for the case wherein a water-soluble cobalt (II) compound is employed as a color indicator.

When the test strip of the invention is impregnated with a chloride-containing specimen, a $Cl^-$ ion in the specimen is first reacted with the $Ag^+$ ion contained in the upper layer to form white precipitates of AgCl.

Since $Ag^+$, $Cl^-$ and $Co^{2+}$ ions can pass through the separation membrane, concentration of the chlorine ion can be judged by the presence or absence of the color that is developed if they reach the carrier impregnated with a water-soluble thiocyanate.

1) In case where $Cl^-$ concentration is smaller than or equal to the difference in concentration between $Ag^+$ and $SCN^-$

The $Ag^+$ remaining unreacted with $Cl^-$ reaches the carrier where it is reacted with all of the $SCN^-$ to form white precipitates. As the $SCN^-$ is entirely consumed, there will be no color developed.

2) In case where $Cl^-$ concentration is larger than the difference in concentration between $Ag^+$ and $SCN^-$

As all of the $Ag^+$ is consumed by the $Cl^-$ or if the $Ag^+$ remains after the reaction with $Cl^-$ and reaches the carrier, it cannot entirely consume the $SCN^-$, a part of the $SCN^-$ remains unconsumed, which will react with the color indicator to develop color.

Thus, the presence or absence of color reaction indicates whether the concentration of $Cl^-$ is higher or lower than the difference in concentration between $Ag^+$ and $SCN^-$.

As two or more of the test strips having a variety of difference in concentrations between $Ag^+$ and $SCN^-$ are fixed on the test device of the invention, amount of the chlorine ion can semi-quantitatively be determined from the number of the test strips which have developed colors.

As the degree of color development is proportional to the concentration of $Cl^-$ when proportions of the reagents incorporated are adequately adjusted, the concentration of $Cl^-$ can also be measured by comparison with the standard colors.

The invention will be described in more detail with reference to Examples and Test Examples.

Example 1

A filter paper was immersed in Solution 1 shown below in Table 1 and then dried. The dried filter paper was then immersed in Solution 2 followed by drying. The same procedures were followed also with Solution 3. A yellow test strip was obtained.

## Table 1

| Immersion solution | Reagent (Concentration w/v%) | Solvent |
|---|---|---|
| Solution 1 | Ammonium thiocyanate (0.8) | Acetone |
| Solution 2 | Hydroxypropylmethyl-cellulose (0.4) | Methylene chloride: isopropyl alcohol (3:2) |
| Solution 3 | Ferric chloride (4.0) Silver nitrate (4.0) | Methyl cellosolve |

4

Example 2

Test strips 1-4 were prepared by the same procedures as described in Example 1 with silver nitrate concentrations respectively set as shown in Table 2 below and the same composition of the other reagents as in Example 1.

Table 2

| Test strip No. | Silver nitrate concentration w/v% |
|---|---|
| 1 | 4.5 |
| 2 | 4.0 |
| 3 | 3.5 |
| 4 | 3.0 |

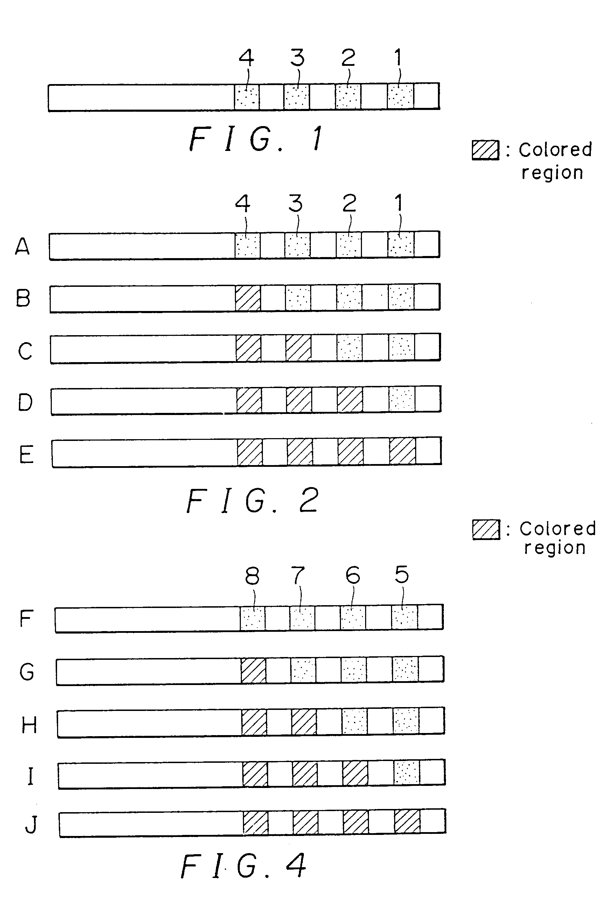

Then, the test strips 1-4 prepared as above were put on a plastic support as shown in Fig. 1. There was obtained a test device for the measurement of chlorides.

Test Example 1

The test devices obtained in Example 2 were respectively immersed into five specimens A-E having different NaCl concentrations, and each test strip was observed for development of the color (red).

The results are shown in Fig. 2 which indicates that no color was developed at all with Specimen A, and the color was developed respectively in Test Strip 4 only with Specimen B, Test Strips 3 and 4 with Specimen C, Test Strips 2-4 with Specimen D and all of the test strips with Specimen E.

It was concluded from the above results that the NaCl concentration is below 0.8 w/v% in Specimen A, from 0.8 w/v% to below 0.93 w/v% in Specimen B, from 0.93 w/v% to below 1.07 w/v% in Specimen C, from 1.07 w/v% to below 1.2 w/v% in Specimen D and at or above 1.2 w/v% in Specimen E.

Example 3

Test strips were prepared in accordance with the procedures described in Example 1 using Solution 1 to Solution 4 shown in Table 3 below.

Table 3

| Immersion solution | Reagent (Concentration w/v%) | Solvent |
|---|---|---|
| Solution 1 | Ammonium thiocyanate (0.7) Sodium alginate (0.5) | Distilled water |
| Solution 2 | Methyl cellulose (0.5) | Methylene chloride-methanol (20:1) |
| Solution 3 | Silver nitrate (5.0) Polyacrylic acid (0.5) | Methyl cellosolve |
| Solution 4 | Cobalt (II) nitrate (2.0) Ammonium tetra-butylnitrate (5.0) Tributyl phosphate (2.0 v/v%) Nonion HS 210 (1.0 v/v%) | Methylene chloride |

Test Example 2

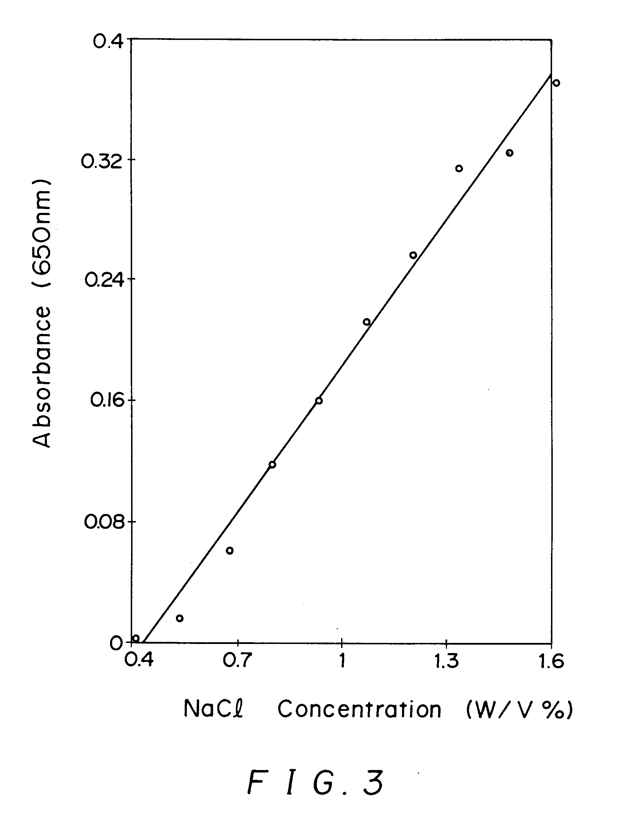

In order to investigate the color reaction in the test strips obtained in Example 3, color development of the test strips were examined by the measurement of absorbance after immersed in several specimens having different NaCl concentrations. The results are shown in Fig. 3

The graph in Fig. 3 represents reflection intensity of the test strip at 650 nm calculated in terms of the absorbance (the axis of ordinate) plotted against NaCl concentration (w/v%) (the axis of abscissas). As seen from the graph, the NaCl concentration is approximately proportional to the absorbance of the test strip. Accordingly, the NaCl concentration can be quantitatively determined by the use of a color table or a reader for the test strips.

Example 4

Test strips were prepared by the same procedures as described in Example 1 using Solution 1 to Solution 4 shown in Table 4 below.

## Table 4

| Immersion solution | Reagent (Concentration w/v%) | Solvent |
|---|---|---|
| Solution 1 | Ammonium thiocyanate (1.0) Sodium alginate (0.5) | Distilled water |
| Solution 2 | Methyl cellulose (0.5) | Methylene chloride-methanol (20:1) |
| Solution 3 | Silver nitrate (5.0) Polyacrylic acid (0.5) Cobalt (II) nitrate (2.0) | Methyl cellosolve |
| Solution 4 | Ammonium tetrabutyl-nitrate (5.0) Tributyl phosphate (2.0 v/v%) Nonion HS 210 (1.0 v/v%) Polyvinylpyrrolidone (0.1) | Methylene chloride |

Example 5

Silver nitrate concentration was as shown in Table 5 below, and composition of the other reagents was the same as in Example 4. Test strips 5-8 were prepared by the same procedures as described in Example 1.

Table 5

| Test strip No. | Silver nitrate concentration (w/v%) |
|---|---|
| 5 | 7 |
| 6 | 6 |
| 7 | 5 |
| 8 | 4 |

Then, the test strips 5-8 obtained as above were put on a plastic support at regular intervals. There was obtained a test device for the measurement of chlorides.

6

Test Example 3

The test devices obtained in Example 5 were respectively immersed into five specimens (F-J) having different NaCl concentrations, and each test strip was observed for development of the color (blue).

The results are shown in Fig. 4 which indicates that no color was developed at all with Specimen F, and the color was developed respectively in Test Strip 8 only with Specimen G, Test Strips 7 and 8 with Specimen H, Test Strips 6-8 with Specimen I and all of the test strips with Specimen J.

It was concluded from the above results that the NaCl concentration is below 0.4 w/v% in Specimen F, from 0.4 w/v% to below 0.53 w/v% in Specimen G, from 0.53 w/v% to below 0.67 w.v% in Specimen H, from 0.67 w/v% to below 0.8 w/v% in Specimen I and at or above 0.8 w/v% in Specimen J.

Industrial Applicability

The present invention is concerned with a test strip comprising a layer of a water-soluble silver salt and a color indicator put via a separation-membrane layer on a carrier carrying a water-soluble thiocyanate. According to the invention chloride concentration in a specimen can be measured without titration but only by immersing a test strip in a liquid specimen to judge whether or not color is developed in the test strip.

Moreover, the invention is concerned with a test device having two or more of the above-mentioned test strips fixed on a support, each of which is different in silver nitrate concentrations. The concentration of chlorides in liquid specimens can be more quickly measured from the number of the test strips with color developed.

The quantitative assay according to the invention is used for the measurement of a chloride in the form of liquid specimens such as plasma, plasma substitutions, urine, amino acid and/or vitamin-containing transfusions, fruit juice, wine, beer, soup, miso soup and food extracts. Therefore, the invention is applicable in the fields of medical industry and food industry.

Brief Description of the Drawing

Fig. 1 is an illustration of the test device for the measurement of a chloride according to the invention.

Fig. 2 is an illustration indicating the presence or absence of color development for the test strips (1-4) in Test Example 1.

Fig. 3 is a graph indicating the absorbance of the test strip $\underline{vs}$ the concentration of NaCl in Test Example 2.

Fig. 4 is an illustration indicating the presence or absence of color development for the test strips (5-8).

In Fig. 1, Fig. 2 and Fig. 4 the numerals 1-8 indicate a test strip, respectively.

## Claims

1. A test strip for the measurement of a chloride comprising a carrier on which a water-soluble thiocyanate is carried, a separation-membrane layer mounted on the upper surface of said carrier and a water-soluble silver-salt and color-indicator layer mounted on the upper surface of said separation-membrane layer, said separation-membrane layer being capable of preventing the permeation of the water-soluble silver salt and the color indicator when they are in dry and solid state and allowing the silver salt and the color indicator to permeate when they are in ionized or dissolved state.

2. A test strip according to Claim 1 wherein the carrier is a filter paper.

3. A test strip according to Claim 1 or 2 wherein said water-soluble silver-salt and color-indicator layer is composed of a water-soluble silver salt layer and a color-indicator layer.

4. A test strip according to any one of Claims 1-3 wherein said water-soluble thiocyanate is soluble in an organic solvent.

5. A test strip according to any one of Claims 1-4 wherein said separation membrane layer comprises substantially a macromolecular compound.

6. A test strip according to Claim 5 wherein the macromolecular compound is hydroxypropylmethylcellulose or methylcellulose.

7

7. A test device for the measurement of a chloride comprising a support and two or more test strips fixed on said support which comprise a carrier carrying a water-soluble thiocyanate and a separation-membrane layer mounted on the upper surface of said carrier and a water-soluble silver-salt and color indicator layer mounted on the upper surface of said separation-membrane layer, said separation-membrane layer being capable of preventing the permeation of the water-soluble silver salt and the color indicator when they are in dry and solid state and allowing the water-soluble silver salt and the color indicator to permeate when they are in ionized or dissolved state and a concentration of the water-soluble silver salt and/or the water-soluble thiocyanate in the test strips being different one another.

**Patentansprüche**

1. Teststreifen zur Chloridbestimmung mit einem Träger mit einem darauf befindlichen wasserlöslichen Thiocyanat, einer auf der Oberseite des Trägers vorgesehenen Trennmembranschicht und einer auf der Oberseite der Trennmembranschicht befindlichen Schicht mit einem wasserlöslichen Silbersalz und einem Farbindikator, wobei die Trennmembranschicht den Durchtritt des wasserlöslichen Silbersalzes und des Farbindikators in trockenem und festem Zustand zu verhindern vermag und das Silbersalz und den Farbindikator in ionisiertem oder gelöstem Zustand durchtreten läßt.

2. Teststreifen nach Anspruch I, wobei der Träger aus einem Filterpapier besteht.

3. Teststreifen nach Anspruch 1 oder 2, wobei die Schicht mit dem wasserlöslichen Silbersalz und dem Farbindikator aus einer wasserlöslichen Silbersalzschicht und einer Farbindikatorschicht aufgebaut ist.

4. Teststreifen nach einem der Ansprüche 1 bis 3, wobei das wasserlösliche Thiocyanat in einem organischen Lösungsmittel löslïch ist.

5. Teststreifen nach einem der Ansprüche 1 bis 4, wobei die Trennmembranschicht im wesentlichen aus einer makromolekularen Verbindung besteht.

6. Teststreifen nach Anspruch 5, wobei die makromolekulare Verbindung aus Hydroxypropylmethylcellulose oder Methylcellulose besteht.

7. Testvorrichtung zur Chloridbestimmung mit einem Schichtträger und zwei oder mehreren daran befestigten Teststreifen, umfassend einen Träger mit einem darauf befindlichen wasserlöslichen Thiocyanat, einer auf der Oberseite des Trägers vorgesehenen Trennmembranschicht und einer auf der Oberseite der Trennmembranschicht befindlichen Schicht mit einem wasserlöslichen Silbersalz und einem Farbindikator, wobei die Trennmembranschicht den Durchtritt des wasserlöslichen Silbersalzes und des Farbindikators in trockenem und festem Zustand zu verhindern vermag und das wasserlösliche Silbersalz und den Farbindikator in ionisiertem oder gelöstem Zustand durchtreten läßt und wobei die Konzentration des wasserlöslichen Silbersalzes und/oder des wasserlöslichen Thiocyanats in den Teststreifen voneinander verschieden ist (sind).

**Revendications**

1. Bandelette de détermination pour la mesure d'un chlorure, comprenant un support portant un thiocyanate hydrosoluble, une couche formant membrane de séparation disposée sur la surface supérieure dudit support et une couche de sel d'argent hydrosoluble et d'indicateur coloré disposée sur la surface supérieure de ladite couche formant membrane de séparation, ladite couche formant membrane de séparation étant capable d'empêcher la perméation du sel d'argent hydrosoluble et de l'indicateur coloré lorsqu'ils sont à l'état sec et solide et de permettre la perméation du sel d'argent et de l'indicateur coloré lorsqu'ils sont à l'état ionisé ou dissous.

2. Bandelette de détermination selon la revendication 1, dans laquelle le support est un papier-filtre.

3. Bandelette de détermination selon la revendication 1 ou 2, dans laquelle ladite couche de sel d'argent hydrosoluble et d'indicateur coloré est constituée par une couche de sel d'argent hydrosoluble et par une couche d'indicateur coloré.

**4.** Bandelette de détermination selon l'une quelconque des revendications 1 à 3, dans laquelle ledit thiocyanate hydrosoluble est soluble dans un solvant organique.

**5.** Bandelette de détermination selon l'une quelconque des revendications 1 à 4, dans laquelle ladite couche formant membrane de séparation comprend essentiellement un composé macromoléculaire.

**6.** Bandelette de détermination selon la revendication 5, dans laquelle le composé macromoléculaire est l'hydroxypropylméthylcellulose ou la méthylcellulose.

**7.** Dispositif de détermination pour la mesure d'un chlorure, comprenant un support et deux ou plus de deux bandelettes de détermination fixées sur ledit support, qui comprennent un support portant un thiocyanate hydrosoluble, une couche formant membrane de séparation disposée sur la surface supérieure dudit support et une couche de sel d'argent hydrosoluble et d'indicateur coloré disposée sur la surface supérieure de ladite couche formant membrane de séparation, ladite couche formant membrane de séparation étant capable d'empêcher la Perméation du sel d'argent hydrosoluble et de l'indicateur coloré lorsqu'ils sont à l'état sec et solide et de permettre la perméation du sel d'argent hydrosoluble et de l'indicateur coloré lorsqu'ils sont à l'état ionisé ou dissous, et les concentrations en sel d'argent hydrosoluble et/ou en thiocyanate hydrosoluble des bandelettes de détermination étant différentes les unes des autres.

F I G . 1

☒ : Colored region

F I G . 2

☒ : Colored region

F I G . 4

F I G. 3